# EUROPEAN PATENT SPECIFICATION

(11) **EP 2 873 429 B1**
(45) Date of publication and mention of the grant of the patent: **06.12.2017**
(21) Application number: 13817500.5
(22) Date of filing: 08.07.2013
(51) Int. Cl.: A61L 27/36, A61L 27/38, C12N 5/071

(54) **DECELLULARIZATION METHOD FOR PREPARING EXTRACELLULAR MATRIX SUPPORT MATERIAL**
DEZELLULARISIERUNGSVERFAHREN ZUR HERSTELLUNG EINES TRÄGERMATERIALS FÜR EXTRAZELLULÄRER MATRIX
PROCÉDÉ DE DÉCELLULARISATION POUR LA PRÉPARATION D'UNE MATIÈRE DE SUPPORT DE MATRICE EXTRACELLULAIRE

(30) Priority: 10.07.2012 CN 201210237911
(43) Date of publication of application: 20.05.2015
(73) Proprietor: SHANGHAI MICROPORT CARDIOFLOW MEDTECH CO., LTD., Shanghai 201203 (CN)
(72) Inventor: DONG, Jiaoming, Shanghai 201203 (CN); LI, Yu, Shanghai 201203 (CN); CHEN, Dakai, Shanghai 201203 (CN); CHEN, Yemeng, Shanghai 201203 (CN); WANG, Jing, Shanghai 201203 (CN); MU, Yuanshan, Shanghai 201203 (CN); FANG, Yuan, Shanghai 201203 (CN); CHEN, Guoming, Shanghai 201203 (CN); ZHANG, Xiaoyi, Shanghai 201203 (CN); YU, Chengyun, Shanghai 201203 (CN); LUO, Qiyi, Shanghai 201203 (CN)
(74) Representative: Patentship Patentanwaltsgesellschaft mbH
(86) International application number: PCT/CN2013/079006
(87) International publication number: WO 2014/008844

(56) References cited:
- WO-A2-02/40630
- WO-A2-2011/019822
- WO-A2-2011/042794
- CN-A- 102 671 242
- US-A1- 2003 035 843
- US-A1- 2010 222 877

## Description

### TECHNICAL FIELD

The present invention relates to the field of tissue-engineered materials. More particularly, the invention is directed to a decellularization method for preparing extracellular matrix (ECM) scaffold.

### BACKGROUND

A tissue is composed of cells and extracellular matrix (ECM). As antigens, the cellular components therein can cause inflammation or immune rejection when recognized by the host. The ECM is a complex of structural and functional proteins and is usually protected and well tolerated across different species. ECM-based biological scaffolds have found extensive use in the medical field of surgical reconstruction and regeneration of tissues and organs, such as cardiac valves, vessels, skin, nerves, tendons, small intestinal submucosa, etc. The ECM is secreted by cells in organs and tissues and interacts dynamically with the cells. It can reflect changes in the microenvironments in real time and plays an important role in the transmigration, differentiation and proliferation of the cells. The ECM has a three-dimensional structure that resembles the cells' natural growth environment in the body. Therefore, it can not only function as a scaffold but also contains a wide range of growth factors which are important facilitators for tissue repair and reconstruction. It remains active even after undergoing a decellularization treatment and is thus an ideal environment for cell growth.

Decellularizing an allogeneic or xenogeneic connective tissue (for example, pericardium, valve, skin, vessel or small intestinal submucosa) with a known conventional method will result in a so-called acellular extracellular matrix. During the decellularization, cells that can cause tissue rejection are removed, remaining the complex structure and key biochemical contents of the original tissue. The composition and ultramicrostructure of the resulting acellular matrix depend directly on what decellularization method is used. Different decellularization methods will lead to acellular matrices with different compositions and ultramicrostructures, which ultimately impact the host's responses after implantation.

There are currently a variety of methods for preparing acellular matrices, mainly including physical, chemical and biological approaches. Most of the physical approaches achieve decellularization by means of physical treatments such as freezing and thawing, high hydraulic pressures, ultrasonic waves and electric shock. The basic principle of such treatments is to damage membrane structures of tissue cells and to thus cause adverse biochemical reactions therein. If the treatments are continued, the cells will be ultimately killed, and the tissue can be subsequently decellularized by washing with a solution and removal of nucleic acids and lipids. The chemical methods are to break up cells by chemical agents so as to enable their removal. Particular chemicals, such as acids, alkalis and detergents, can penetrate all layers of a tissue and cause the death and destruction of cells by dissolving phosopholipids that make up cell membrane bilayers and even destroying cell membrane proteins. This is followed by shaking, rinsing and other procedures for washing away cell debris and antigenic substances. In most biological approaches, enzymes are used to lyse cells. For example, trypsin, a member of the serine protease family, is usually used in such methods. Trypsin can selectively hydrolyze protein peptide chains formed by the carboxyl groups of lysine or arginine. Under the action of trypsin, hydrolysis of intercellular proteins and hence cell dissociation will be caused.

Chinese Patent Application No. 201110134511.9 relates to a method of preparing a homologous acellular dermal matrix, in which sodium dodecyl sulfate is used for decellularization. Although this method can enable a short processing time, the sodium dodecyl sulfate is not conducive to in-vivo implantation because it can cause considerable damage to the matrix structure and is significantly toxic. Chinese Patent Application No. 200510002464.7 is directed to a method of decellularization for a xenogeneic cardiovascular graft and Chinese Patent Application No. 200910076674.9 concerns a vascular matrix from a decellularized vascular tissue and a method of preparing the matrix. Both of these two methods involve the use of trypsin, with different concentrations and treatment times, though. The used trypsin is bound to cause degradation of the resulting scaffolds, possible to different extents between the methods, and thus impair their structures and alter their compositions. A tissue typically swells by about 30% after being treated with trypsin. US2010/222877 discloses a method of decellularization comprising subjecting a harvested tissue to a reciprocating osmotic shock sequence, a detergent wash, a RNA-DNA extraction, an enzyme treatment, and an organic solvent extraction. Suitable detergents include e.g. octyl-b-D-glucopyranoside, decyl a-D-glucopyranoside, or n-undecyl b-D-glucopyranoside.

While conventionally used detergents are capable of removing cells during decellularization treatments, their residuals are a cause of cytotoxicity and potential immunogenicity [Non-patent Reference Documents 2 and 3]. In order to avoid such toxicities, it is necessary to remove the detergent residuals or substitute them with nontoxic reagents. Additionally, as the large number of existing detergent removal approaches are all associated with high process complexity and inadequacy, there exists a need for a novel, nontoxic, degradable substituting reagent that is capable of complete decellularization of a target organic tissue. The use of environmentally friendly, biodegradable, completely nontoxic detergents is of great significance in the preparation of acellular matrices.

n-Octyl-beta-D-glucopyranoside (OGP) is a new "green" surfactant with favorable performance. It possesses the properties of both non-ionic and anionic surfactants, and offers a wide variety of advantages such as low surface tension, high activity, strong detergency, ability to produce abundant, fine, stable foams, no skin irritability, high biodegradability, non-toxicity and no environmental pollution. Studies show that [Non-patent Reference Document 1], by taking its advantage of high surface activity, OGP is able to destroy bacterial cell walls and exhibits a strong antibacterial activity against a broad spectrum of bacteria, including both Gram-positive and -negative bacteria. Therefore, it can serve as an ideal mild nonionic surfactant.

### SUMMARY OF THE INVENTION

It is an objective of the present invention to provide a decellularization method for preparing an extracellular matrix (ECM) scaffold. The method is capable of completely removing cells and cell debris on the surface of and within an organic tissue, thereby forming an ECM with desirable mechanical and biological properties and thus resulting in a scaffold suitable to be used in tissue engineering.

The present invention is directed to a decellularization method for preparing an ECM scaffold, which includes the steps of:
a. performing a pretreatment on a target organic tissue;
b. placing the pretreated organic tissue in a hypotonic solution containing glucopyranoside and shaking to destroy cell membrane structures in the organic tissue and extracting lipids and proteins of the membranes;
c. placing the organic tissue treated by step b in a nuclease solution to degrade DNA and/or RNA components in cells thereof; and
d. rinsing the resulting ECM scaffold.

According to the present invention, the target organic tissue may be a xenogeneic, allogeneic, or autologous organic tissue including one of a bovine pericardium, a porcine pericardium, a heart valve, skin, a vessel, a small intestinal submucosa and a nerve. In general terms, organic tissues rich in collagen or elastic fibers are suitable to be decellularized by this method.

According to the present invention, the pretreatment may include subjecting the target organic tissue to routine rinsing, disinfection and dissection processes, performed at a temperature in the range of 0 °C to 37 °C and using a normal saline containing antibiotics, or phosphate buffered saline with a pH of 6.8-8.6 and containing antibiotics, or a D-Hank's solution with a pH of 6.8-8.6 and containing antibiotics (Hank's solutions are commonly used balanced salt solutions (BSSs) and also basic solutions for tissue culturing, whilst the D-Hank's solution (Gibco) is a Hank's solution that is free of calcium and magnesium ions). The antibiotics may include the antibiotics of one of two antibiotic groups. The antibiotics in either group are generally used in combination. One of the two antibiotic groups is consisted of amikacin, flucytosine, vancomycin and chloromycetin, and the other is consisted of penicillin, streptomycin sulfate and amphotericin B. For example, the antibiotics are preferably pen-strep (Gibco).

According to the present invention, the glucopyranoside may be one of n-octyl-β-D-glucopyranoside (OGP), octyl-β-D-thioglucopyranoside, nonyl glucopyranoside, decyl glucopyranoside, and dodecyl glucopyranoside, or a combination thereof (the combination can be collectively referred to as alkyl glucopyranosides), with OGP being preferred.

According to the present invention, the glucopyranoside may be present in the solution at a concentration of lower than 70% (w/v), i.e., a ratio of weight/volume of lower than 70 g/100 ml, preferably 0.1-10% (w/v), i.e., a ratio of weight/volume of 0.1-10 g/100 ml, and more preferably 0.5-5% (w/v). The solution containing glucopyranoside may have a temperature of 1-40 °C, preferably 3-8 °C, and a pH of 5-12, preferably 6.8-8.6. The treatment time can be from 30 minutes to 96 hours, preferably 6-48 hours.

Shaking the organic tissue in the hypotonic solution may include shaking the tissue in a sterile 0.01 M Tris-HCl buffer (pH 6.8-8.6), at a temperature of 1-40 °C (preferably 3-8 °C), for from 30 minutes to 96 hours (preferably 6-48 hours), at a speed of 50-360 rpm, preferably 100-250 rpm.

The nuclease solution may be prepared by adding 100-10000 U/ml (preferably1000-8000 U/ml) of DNase and RNase to a sterile 0.02-0.05 M Tris-HCl buffer (pH 6.8-8.6) containing 0.15 M of NaCl and 1-5 mM of MgCl₂. After that, the organic tissue can be placed in the nuclease solution and shaken at a temperature of 37°C and a speed of 60-360 rpm (preferably 100-250 rpm) for 12-72 hours.

The rinsing may include rinsing the resulting acellular matrix in a routine manner at a temperature of 1-25°C for 6-48 hours, with a normal saline, or a phosphate buffered solution with a pH of 6.8-8.6, or a D-Hank's solution with a pH of 6.8-8.6, containing antibiotics.

The acellular matrix obtained by the above method can be assessed in accordance with the standards and methods described below.
1. Residual cells. The acellular matrix is fixed in 10% neutral formalin, embedded in paraffin and sliced into 0.4 micron thick pieces. After being subsequently deparaffinized by xylene, desiccated serially in ethanol and stained with hematoxylin and eosin, the pieces are observed for residual cells therein.
2. Fibrous structures. The acellular matrix is fixed in 10% neutral formalin, embedded in paraffin and sliced into 0.4 micron thick pieces. After being subsequently deparaffinized by xylene, desiccated serially in ethanol and stained with Movat pentachrome, the pieces are observed for distribution and structural variation of collagen fibers, elastic fibers and glycoproteins in the matrix.
3. Measurement of DNA content of the acellular matrix. The tissue is ground and split to produce samples. DNA from a sample is extracted by using a PureLink genomic DNA mini kit and measured using a PicoGreen® dsDNA assay kit (Invitrogen). The DNA content is calculated based on an absorbance measured at a wavelength of 545 nm.
4. Measurement of water content of the acellular matrix. An undried sample is sandwiched between two pieces of dry filter paper and an object with a weight of 50g is loaded thereon for 30 seconds. Wet weight of the sample is subsequently measured. The sample is frozen in vacuum for 24 hours and its dry weight is then measured. The water content is calculated according to: water content = (wet weight - dry weight)/dry weight × 100%.
5. Residual cells on surface. The acellular matrix is fixed in a 2.5% glutaraldehyde solution, desiccated serially in ethanol, dried, sprayed with gold in vacuum and observed under a scanning electron microscope (SEM) for residual cells and distribution of fibers on its surface.
6. Measurement of collagen content. The acellular matrix is placed in 6M HCl at 106°C for 24 hours for digestion. The products of the digestion are then further digested with chloramine-T at 20°C for 20 minutes, heated in a perchloric aldehyde acid at 60 °C for 15 minutes in a water bath, and measured at 550 nm for the absorbance. Hydroxyproline is quantified by the absorbance and the collagen content is calculated on the basis of a percentage of about 12.7% of the hydroxyproline in the collagen.
7. Measurement of elastin content. The acellular matrix is boiled at 100°C in a 0-2 mmol/l oxalic acid solution for 60 minutes and centrifuged, and the supernatant is saved. This process is further repeated twice and all the saved supernatants are combined. The elastin content (/dry weight) is measured using a Fastin elastin assay kit (Biocolor).
8. Measurement of tensile strength of the acellular matrix. The acellular matrix is trimmed into plate-shaped specimens, and the specimens are subsequently drawn to break on a universal testing machine. The tensile strength, which represents the matrix's ability to withstand tensile forces, is calculated as the ratio of the recorded maximum tension force to the cross-sectional area of the specimens.
9. Measurement of breaking elongation of the acellular matrix. The acellular matrix is trimmed into plate-shaped specimens. The specimens are subsequently drawn to break on a universal testing machine and their lengths at the time of breaking are recorded. The breaking elongation, which represents the matrix's deformability, is calculated as the percentage of the specimens' elongated length to their original length.
10. Measurement of elastic modulus of the acellular matrix. The acellular matrix is trimmed into plate-shaped specimens, and the specimens are subsequently drawn to break on a universal testing machine to derive their stress-strain relationship. The stress-strain relationships are then plotted and subjected to linear fitting, and the elastic modulus, which represents the matrix's ability to withstand elastic deformation, is obtained as the slope of the fitted line.
11. Test on in-vitro cytotoxicity of the acellular matrix. Extract assay and MTT assay are performed in accordance with the protocols described in Chinese national standards GB16886.5-2003 and GB/T 14233.2-2005_8 to determine the in-vitro cytotoxicity of the acellular matrix.

The glucopyranoside according to the present invention is synthesized from renewable natural fatty alcohols (fatty alcohols as used herein refer to those containing 8 to 22 carbon atoms, including natural and synthetic fatty alcohols. Natural fatty alcohols are obtained from natural animal and vegetable fats and oils, such as coconut oil, palm oil and beef tallow. Alcohols resulting from the reduction of fatty acids produced by the hydrolysis of such fats and oils are collectively called natural fatty alcohols.) and glucose. It is a relatively all-round new nonionic surfactant that possesses properties of both non-ionic and anionic surfactants. Thanks to its high surface activity, bio-safety and biocompatibility, it is the internationally-recognized, most preferred "green" functional surfactant. In addition, it is highly soluble, mild, capable of degreasing, less irritant to skin, nontoxic and easy to be washed off. Further, it has capacities of disinfection and mitigating irritation as well as other favorable properties. Glucopyranosides generally remain stable in strong alkalis, strong acids and high-concentration electrolytes, have less corrosive effect, and are readily biodegradable and will thus not cause pollution to the environment. They are mild in nature and have almost no damage to ultramicrostructures and functional proteins of ECMs. In addition, they are nontoxic, readily degradable and harmless to human body. In order to facilitate the decellularization of glucopyranosides, physical approaches can be jointly used to treat the target organic tissue. In the method of the present invention, the decellularization of the glucopyranoside may be enhanced primarily by the physical approaches: hypotonic solution immersion, oscillation and temperature control.

According to the present invention, the joint use of the glucopyranoside, hypotonic solution and nuclease can efficiently remove cellular components in a target organic tissue and circumvent the drawbacks associated with the use of conventional surface surfactants such as toxicity, inadequate decellularization or damages to the resulting ECM, thereby resulting in a desirable tissue-engineering scaffold with significantly reduced immunogenicity, low cytotoxicity and high biocompatibility.

### BRIEF DESCRIPTION OF DRAWINGS

Features of the invention will become apparent from the following description, taken in conjunction with the accompanying drawings. It is apparent that what are set forth in the drawings are merely several specific embodiments described in this specification. The features of the present invention include, but are not limited to, those set forth in the drawings.
FIG. 1 is a microscopic image (HE, ×200) of a fresh porcine pericardium which contains cells.
FIG. 2 is a microscopic image (HE, ×200) of the fresh porcine pericardium after being decellularized, illustrating that after treated by a decellularization method according to the present invention, all cellular structures in the tissue have disappeared, indicating a capability of complete decellularization of the method.
FIG. 3 is another microscopic image (Movat Pentachrome, ×400) of the fresh porcine pericardium.
FIG. 4 is another microscopic image (Movat Pentachrome, ×400) of the fresh porcine pericardium after being decellularized, illustrating that, after treated by the decellularization method according to the present invention, collagen fibers in the tissue still keeps an orderly arrangement and a compact, parallel-corrugated configuration without obvious rupture, elastic fibers therein are still clearly distinguishable, and there is no obvious swelling in the tissue.
FIG. 5 is still another microscopic image (800 ×) of the fresh porcine pericardium.
FIG. 6 is still another microscopic image (800 ×) of the fresh porcine pericardium after being decellularized, illustrating that, after treated by the decellularization method according to the present invention, collagen fibers in the tissue still keeps an orderly arrangement and a compact, continuous, parallel-corrugated configuration without obvious rupture.

### DETAILED DESCRIPTION

For a better understanding of the present invention, certain preferred features of the present invention are described in the following Embodiments. The description is provided merely for exemplary illustration of the features and advantages of the invention but not for limiting the scope of the invention.

### EMBODIMENT 1

1) Materials. Porcine pericardia were obtained from a local slaughterhouse, where hearts were harvested from healthy adult pigs and the pericardiums were peeled off the hearts, with a warm ischemia time not greater than 2 hours. After blood clots were removed by repeated rinsing with a phosphate buffered saline (PBS), the pericardia were placed in a preservative fluid and transported to the laboratory. After the surrounding fat was trimmed off under sterile conditions, portions of anterior walls of the pericardia, which were not damaged and uniform in thickness, were dissected into 3 cm× 4 cm pieces. The pieces were then thoroughly rinsed in a PBS and stored at 4°C in a sterile PBS containing antibiotics.
2) Decellularization of the porcine pericardia. Six of the 3 cm× 4 cm pericardial pieces were placed in respective six bottles of a 10 mM Tris-HCl buffer containing 1% of OGP (Aladdin Reagent (Shanghai) Co., Ltd) and shaken at a temperature of 4°C and a speed of 150 rpm/min for 24 hours for digestion. The buffer was a double-antibiotic solution containing 1% of streptomycin sulfate (Aladdin Reagent (Shanghai) Co., Ltd, Batch No.: C1208010) and penicillin sodium (Aladdin Reagent (Shanghai) Co., Ltd, Batch No.: 16458). Afterward, the pieces were washed in a sterile PBS (pH 7.30) five times each for 15 minutes and then successively placed in a nuclease solution and shaken at 37°C for 24 hours for further digestion. The nuclease solution was a sterile 50 mM Tris-HCl buffer (pH 7.60) containing 2.5 kU/ml DNase I (Sigma), 7.5 kU/ml RNase (Sigma), 0.15 M NaCl, 2 mM MgCl₂(H₂O)₆, and 1% double-antibiotic.
3) Rinsing. The pericardial pieces were shaken in a sterile double-antibiotic PBS (pH 7.30) for 24 hours at a speed of 150 rpm for rinsing. The rinsed pieces were then successively placed in a sterile double-antibiotic PBS and stored therein at 4°C.

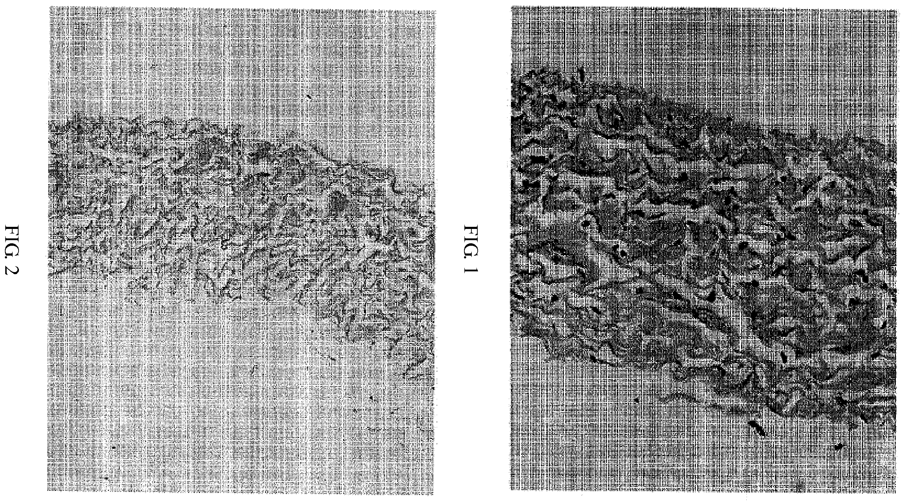

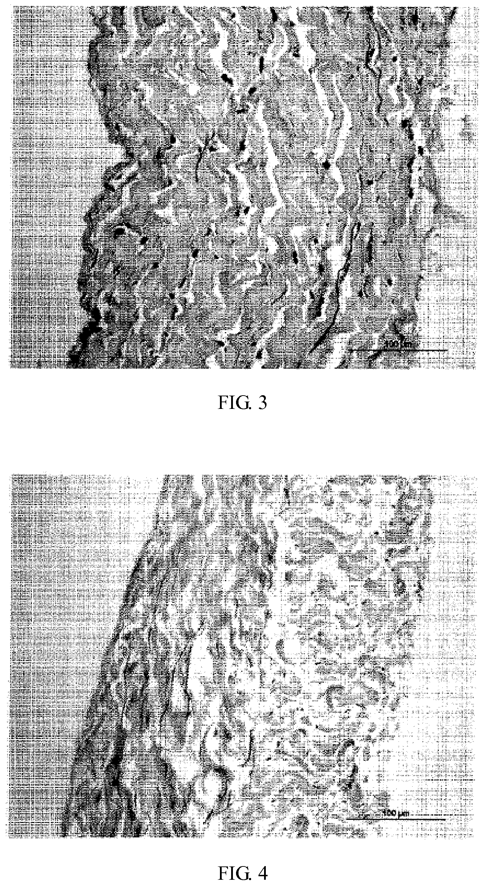

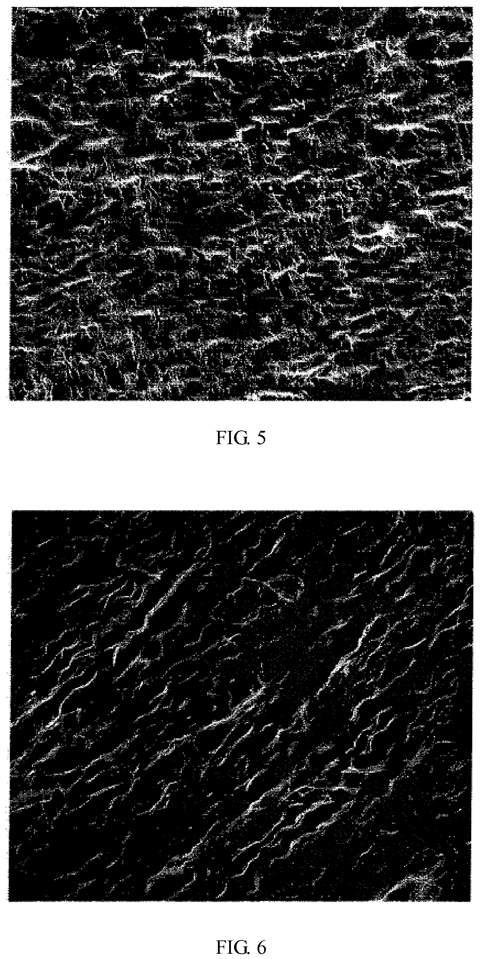

The results were assessed as follows:
1. Residual cells. The decellularized pericardial pieces were shown to have no cellular structure by a regular hematoxylin and eosin staining (see FIG. 2).
2. Fibrous structures. By Movat pentachrome staining, it was observed that, collagen fibers in the decellularized pericardial pieces still kept an orderly arrangement and a compact, parallel-corrugated configuration without obvious rupture and elastic fibers therein were still clearly distinguishable (see FIGS. 4 and 6).
3. Measurement of DNA content of the acellular matrix. With the DNA test kits, the DNA content of the porcine pericardia before the decellularization was measured to be 522.063 ± 46.44 ng per 1 mg dry weight, while the DNA content of the decellularized pericardial pieces was measured to be 5.642 ±1.75 ng per 1 mg dry weight. A significant statistical difference was observed.
4. Measurement of water content of the acellular matrix. The water content of the fresh porcine pericardia was 79.427±0.29%, and the water content of the decellularized pericardial pieces was 81.432±0.74%. No significant statistical difference was found.
5. Residual cells on surface of the matrix. In SEM observations, a great number of cells were found on surface of the porcine pericardia before the decellularization, while orderly fibers without rupture were seen on the decellularized pericardial pieces.
6. Measurement of collagen content. Based on the respective measured hydroxyproline quantities, the collagen content of the porcine pericardia before the decellularization was calculated to be 46.218±1.27%, and that of the decellularized pericardial pieces to be 46.785±0.42%. No significant statistical difference was found.
7. Measurement of elastin content. With the Fastin elastin assay kits, the elastin content of the porcine pericardia before the decellularization was measured to be 5.185±0.005 µg per 1 mg dry weight, and that of the decellularized pericardial pieces to be 4.316±0.001 µg per 1 mg dry weight. No significant statistical difference was found.
8. Measurement of tensile strength of the acellular matrix. On the universal testing machine, the maximum tensile strength of the fresh porcine pericardia was measured to be 14.362±0.82 MPa, and that of the decellularized pericardial pieces to be 13.461±0.55 MPa. No significant statistical difference was found.
9. Measurement of breaking elongation of the acellular matrix. On the universal testing machine, the breaking elongation of the fresh porcine pericardia was measured to be 70.621±5.09%, while the breaking elongation of the decellularized pericardial pieces was measured to be 79.235±2.81%. A significant statistical difference was observed.
10. Measurement of elastic modulus of the acellular matrix. On the universal testing machine, the elastic modulus of the fresh porcine pericardia was measured to be 81.335±4.23 MPa, and that of the decellularized pericardial pieces to be 66.302±6.13 MPa. No significant statistical difference was found. The treated tissue tended to be slightly softer.
11. Test on in-vitro cytotoxicity of the acellular matrix. In the extract and MTT assays, the relative growth rate of the fresh porcine pericardia was determined to be 102.325±1.77% and the cytotoxicity level thereof as Level 0; and the relative growth rate of the decellularized pericardial pieces was determined to be 92.167±1.35% and the cytotoxicity level thereof as Level 1. No significant statistical difference was found.

### EMBODIMENT 2

Pericardial pieces were produced in the same manner as described in embodiment 1. Six of the produced 3 cm× 4 cm pericardial pieces were then placed in respective six bottles of a 2% OGP, 10 mM Tris-HCl buffer and shaken at a temperature of 4°C and a speed of 150 rpm/min for 16 hours for digestion. The buffer was also a double-antibiotic solution containing 1% of the streptomycin sulfate and the penicillin sodium. Subsequently, the pieces were washed in a sterile PBS (pH 7.30) five times each for 15 minutes and then successively placed in a nuclease solution and shaken at 37°C for 24 hours for further digestion. The nuclease solution was also a sterile 50 mM Tris-HCl buffer (pH 7.60) containing 2.5 kU/ml DNase I, 7.5 kU/ml RNase, 0.15 M NaCl, 2 mM MgCl₂(H₂O)₆, and 1% double-antibiotic. The pericardial pieces were subsequently shaken in a sterile double-antibiotic PBS (pH 7.30) for 24 hours at a speed of 150 rpm for rinsing. The rinsed pieces were then successively placed in a sterile double-antibiotic PBS and stored therein at 4°C.

### EMBODIMENT 3

Pericardial pieces were produced in the same manner as described in embodiment 1. Six of the produced 3 cm× 4 cm pericardial pieces were then placed in respective six bottles of a 10 mM Tris-HCl buffer containing 1% of dodecyl glucopyranoside (Aladdin Reagent (Shanghai) Co., Ltd) and shaken at a temperature of 4°C and a speed of 150 rpm/min for 24 hours for digestion. The buffer was also a double-antibiotic solution containing 1% of the streptomycin sulfate and the penicillin sodium. Subsequently, the pieces were washed in a sterile PBS (pH 7.30) five times each for 15 minutes and then successively placed in a nuclease solution and shaken at 37°C for 24 hours for further digestion. The nuclease solution was a sterile 20 mM Tris-HCl buffer (pH 7.60) containing 2.5 kU/ml DNase I, 7.5 kU/ml RNase, 0.15 M NaCl, 2 mM MgCl₂(H₂O)₆, and 1% double-antibiotic. After that, the pericardial pieces were shaken in a sterile double-antibiotic PBS (pH 7.30) for 24 hours at a speed of 150 rpm for rinsing. The rinsed pieces were then successively placed in a sterile double-antibiotic PBS and stored therein at 4°C.

### EMBODIMENT 4

Pericardial pieces were produced in the same manner as described in embodiment 1. Six of the produced 3 cm× 4 cm pericardial pieces were then placed in respective six bottles of a 10 mM Tris-HCl buffer containing 1% of alkyl glucopyranosides (Hebei Shijiazhuang Jinmoer Chemicals, Co., Ltd) and shaken at a temperature of 4°C and a speed of 150 rpm/min for 24 hours for digestion. The buffer was also a double-antibiotic solution containing 1% of the streptomycin sulfate and the penicillin sodium. Subsequently, the pieces were washed in a sterile PBS (pH 7.30) five times each for 15 minutes and then successively placed in a nuclease solution and shaken at 37°C for 24 hours for further digestion. The nuclease solution was a sterile 50 mM Tris-HCl buffer (pH 7.60) containing 2.0 kU/ml DNase I, 8.0 kU/ml RNase, 0.15 M NaCl, 5 mM of MgCl₂(H₂O)₆, and 1% double-antibiotic. After that, the pericardial pieces were shaken in a sterile double-antibiotic PBS (pH 7.30) for 24 hours at a speed of 150 rpm for rinsing. The rinsed pieces were then successively placed in a sterile double-antibiotic PBS and stored therein at 4°C.

The use of biodegradable, nontoxic, non-irritating, non-environmentally polluting octyl-β-D-glucopyranoside for decellularization can achieve complete removal of cells while maintaining the ultramicrostructure and composition of the resulting scaffold.

### REFERENCES

Non-patent Reference Document 1: Ecological properties of alkyl glucosides, Chemosphere, 1997, 35(3): 545-556.
Non-patent Reference Document 2: Detergent decellularization of heart valves for tissue engineering: toxicological effects of residual detergents on human endothelial cells. Artif Organs, 2010, 34(3): 206-210.
Non-patent Reference Document 3: Does Sodium Dodecyl Sulfate Wash Out of Detergent-Treated Bovine Pericardium at Cytotoxic Concentrations? The Journal of Heart Valve Disease, 2009, 18:101-.

## Claims

1. A decellularization method for preparing an extracellular matrix scaffold, comprising the steps of:
a) performing a pretreatment on a target organic tissue;
b) placing the pretreated organic tissue in a hypotonic solution containing glucopyranoside and shaking;
c) placing the organic tissue treated by step b) in a nuclease solution; and
d) rinsing the resulting extracellular matrix scaffold.

2. The method of claim 1, wherein the target organic tissue is a xenogeneic, allogeneic, or autologous organic tissue selected from a group consisting of a bovine pericardium, a porcine pericardium, a heart valve, skin, a vessel, a small intestinal submucosa and a nerve.

3. The method of claim 1 or 2, wherein the pretreatment includes subjecting the target organic tissue to routine rinsing, disinfection and dissection processes performed at a temperature of 0-37 °C and using a normal saline, or a phosphate buffered solution with a pH of 6.8-8.6, or a D-Hank's solution with a pH of 6.8-8.6, containing antibiotics.

4. The method of any of the preceding claims, wherein the glucopyranoside is selected from any one of n-octyl-β-D-glucopyranoside (OGP), octyl-β-D-thioglucopyranoside, nonyl glucopyranoside, decyl glucopyranoside, dodecyl glucopyranoside, or a combination thereof, with OGP being preferred.

5. The method of any of the preceding claims, wherein the glucopyranoside is present in the solution at a concentration of lower than 70% (w/v), preferably 0.1-10% (w/v), and more preferably 0.5-5% (w/v); wherein the solution containing glucopyranoside has a temperature of 1-40 °C, preferably 3-8 °C; wherein the solution containing glucopyranoside has a pH of 5-12, preferably 6.8-8.6; and the shaking continues for 30 minutes to 96 hours, preferably 6-48 hours.

6. The method of any of the preceding claims, wherein shaking the organic tissue in a hypotonic solution includes shaking the organic tissue in a sterile 0.01 M Tris-HCl buffer with a pH of 6.8-8.6, at a temperature of 1-40 °C, preferably 3-8 °C, for 30 minutes to 96 hours, preferably 6-48 hours, at a speed of 50-360 rpm, preferably 100-250 rpm.

7. The method of any of the preceding claims, wherein step c) comprises preparing the nuclease solution by adding 100-10000 U/ml, preferably 1000-8000 U/ml, of DNase and RNase to a sterile 0.02-0.05 M Tris-HCl buffer having a pH of 6.8-8.6 and containing 0.15 M of NaCl and 1-5 mM of MgCl₂; and placing the organic tissue treated by step b) in the nuclease solution and shaking the organic tissue in the nuclease solution at a temperature of 37°C and a speed of 60-360 rpm, preferably 100-250 rpm, for 12-72 hours.

8. The method of any of the preceding claims, wherein step d) comprises rinsing the resulting acellular matrix in a routine manner at a temperature of 1-25°C for 6-48 hours, with a normal saline, or a phosphate buffered solution with a pH of 6.8-8.6, or a D-Hank's solution with a pH of 6.8-8.6, containing antibiotics.

## Patentansprüche

1. Dezellularisierungsverfahren zur Herstellung eines extrazellulären Matrix-Schafottes, mit den Schritten:
a) Durchführen einer Vorbehandlung auf einem organischen Zielgewebe;
b) Platzieren des vorbehandelten organischen Gewebes in einer hypotonischen Lösung, welche Glucopyranosid und Schütteln enthält;
c) Platzieren des durch Schritt b) behandelten organischen Gewebes in einer Nuklease-Lösung; und
d) Spülen des resultierenden extrazellulären Matrix-Schafottes.

2. Verfahren nach Anspruch 1, wobei das organische Zielgewebe ein xenogenes, allogenes oder autologes organisches Gewebe ist, welches aus einer Gruppe ausgewählt ist, welche aus einem Rinder-Pericardium, einem Schweine-Pericardium, einer Herzklappe, Haut, einem Gefäß, einer kleinen Darmsubmukosa und einem Nerv besteht.

3. Verfahren nach Anspruch 1 oder 2, wobei die Vorbehandlung umfasst: Unterziehen des organischen Zielgewebes Routine-Spül-, Desinfektions- und Sektionsverfahren, welche bei einer Temperatur von 0-37° C durchgeführt werden, und Verwenden einer normalen Kochsalzlösung oder einer phosphatgepufferten Lösung mit einem pH-Wert von 6,8-8,6 oder einer D-Hanks Lösung mit einem pH-Wert von 6,8-8,6, enthaltend Antibiotika.

4. Verfahren nach einem der vorstehenden Ansprüche, wobei das Glucopyranosid aus einem von n-Octyl-β-D-glucopyranosid (OGP), Octyl-β-D-thioglucopyranosid, Nonylglucopyranosid, Decylglucopyranosid, Dodecylglucopyranosid, oder einer Kombination davon gewählt ist, wobei OGP bevorzugt ist.

5. Verfahren nach einem der vorstehenden Ansprüche, wobei das Glucopyranosid in der Lösung in einer Konzentration von weniger als 70% (w/v), vorzugsweise 0,1-10% (w/v), und bevorzugter 0,5-5% (w/v) vorhanden ist; wobei die Glucopyranosid enthaltende Lösung eine Temperatur von 1-40° C, vorzugsweise 3-8° C, aufweist; wobei die Glucopyranosid enthaltende Lösung einen pH-Wert von 5-12, vorzugsweise 6,8-8,6, aufweist; und das Schütteln 30 Minuten bis 96 Stunden, vorzugsweise 6-48 Stunden, dauert.

6. Verfahren nach einem der vorstehenden Ansprüche, wobei das Schütteln des organischen Gewebes in einer hypotonischen Lösung umfasst: das Schütteln des organischen Gewebes in einem sterilen 0,01 M Tris-HCl-Puffer mit einem pH-Wert von 6,8-8,6 bei einer Temperatur von 1-40° C, vorzugsweise 3-8° C, für 30 Minuten bis 96 Stunden, vorzugsweise 6-48 Stunden, bei einer Geschwindigkeit von 50-360 U/min, vorzugsweise 100-250 U/min.

7. Verfahren nach einem der vorstehenden Ansprüche, wobei Schritt c) umfasst:
Vorbereiten der Nuklease-Lösung durch Hinzufügen von 100-10000 U/ml, vorzugsweise 1000-8000 U/ml, von DNase und RNase zu einem sterilen 0,02-0,05 M Tris- HCl-Puffer, welcher einen pH-Wert von 6,8-8,6 aufweist und 0,15M von NaCl und 1-5 mM von MgCl₂ enthält; und Platzieren des durch Schritt b) behandelten organischen Gewebes in der Nuklease-Lösung und Schütteln des organischen Gewebes in der Nuklease-Lösung bei einer Temperatur von 37°C und einer Geschwindigkeit von 60-360 U/min, vorzugsweise 100-250 U/min, für 12-72 Stunden.

8. Verfahren nach einem der vorstehenden Ansprüche, wobei Schritt d) umfasst: Spülen der resultierenden azellulären Matrix routinemäßig bei einer Temperatur von 1-25°C für 6-48 Stunden mit einer normalen Kochsalzlösung, oder einer phosphatgepufferten Lösung mit einem pH-Wert von 6,8-8,6, oder einer D-Hanks Lösung mit einem pH-Wert von 6,8-8,6, enthaltend Antibiotika.

## Revendications

1. Procédé de décellularisation pour préparer un échafaudage de matrice extracellulaire, comportant les étapes de:
a) réaliser un prétraitement sur un tissu organique de cible;
b) placer le tissu organique prétraité dans une solution hypotonique comportant du glucopyranoside et agiter;
c) placer le tissu traité dans l'étape b) dans une solution nucléase; et
d) rincer l'échafaudage de matrice extracellulaire obtenu.

2. Procédé selon la revendication 1, dans lequel le tissu organique de cible est un tissu xénogénique, allogénique ou un tissu organique autologue sélectionné dans un groupe comprenant un péricarde bovin, un péricarde de porc, une valvule cardiaque, de la peau, un vaisseau, une petite sous-muqueuse intestinale et un nerf.

3. Procédé selon la revendication 1 ou 2, dans lequel le prétraitement comporte l'étape de soumettre le tissue organique de cible à un rinçage de routine, procédé de désinfection et de dissection réalisés à une température de 0-37 °C et en utilisant une solution physiologique, ou une solution de tampon phosphate ayant un pH de 6.8-8.6, ou une solution de D-Hank ayant un pH de 6.8-8.6, comportant des antibiotiques.

4. Procédé selon l'une des revendications précédentes, dans lequel le glucopyranoside est sélectionné parmi l'un des n-octyle-β-D-glucopyranoside (OGP), octyle-β-D-thioglucopyranoside, nonyl glucopyranoside, décyle glucopyranoside, dodécyle glucopyranoside, ou une combinaison parmi ceux-ci, le OGP étant préféré.

5. Procédé selon l'une des revendications précédentes, dans lequel le glucopyranoside est présent dans la solution à une concentration inférieure à 70% (w/v), préférablement entre 0.1 et 10% (w/v), et encore plus préférablement entre 0.5 et 5% (w/v); dans lequel la solution comportant le glucopyranoside a une température entre 1 et 40 °C, préférablement entre 3 et 8 °C; dans lequel la solution comportant le glucopyranoside a un pH entre 5 et 12, préférablement entre 6.8 et 8.6; et l'agitation continue pendant 30 minute jusqu'à 96 heures, préférablement pendant 6-48 heures.

6. Procédé selon l'une des revendications précédentes, dans lequel l'étape d'agiter le tissu organique dans une solution hypotonique comporte l'étape d'agiter le tissu organique dans un tampon 0.01 M Tris-HCl stérile ayant un pH de 6.8-8.6, à une température de 1-40 °C, préférablement de 3-8 °C, pendant 30 minutes jusqu'à 96 heures, préférablement pendant 6-48 heures, à une vitesse de 50-360 rpm, préférablement de 100-250 rpm.

7. Procédé selon l'une des revendications précédentes, dans lequel l'étape c) comporte l'étape de préparer la solution nucléase en ajoutant 100-10000 U/ml, préférablement 1000-8000 U/ml, de ADNase et ARNase à un tampon de 0.02-0.05 M Tris-HCl stérile ayant un pH de 6.8-8.6 et comportant 0.15 M de NaCl et 1-5 mM de MgCl₂; et l'étape de placer le tissu organique traité dans l'étape b) dans la solution nucléase et l'étape d'agiter le tissu organique dans la solution nucléase à une température de 37 °C et à une vitesse de 60-360 rpm, préférablement de 100-250 rpm, pendant 12-72 heures.

8. Procédé selon l'une des revendications précédentes, dans lequel l'étape d) comporte l'étape de rincer la matrice acellulaire obtenue de façon routinière à une température de 1-25 °C pendant 6-48 heures, avec une solution physiologique, ou une solution de tampon phosphate avec un pH de 6.8-8.6, ou une solution de D-Hank avec un pH de 6.8-8.6, comportant des antibiotiques.
